Europäisches Patentamt

European Patent Office

Office européen des brevets

(11) Veröffentlichungsnummer: **0 215 811**
**B1**

# (12) EUROPÄISCHE PATENTSCHRIFT

(45) Veröffentlichungstag der Patentschrift:
18.10.89

(21) Anmeldenummer: 86901026.4

(22) Anmeldetag: 12.02.86

(86) Internationale Anmeldenummer:
PCT/CH 86/00018

(87) Internationale Veröffentlichungsnummer:
WO 86/04919 (28.08.86 Gazette 86/19)

(51) Int. Cl.⁴: **C 12 M 3/00, C 12 N 5/00, A 01 G 7/00**

(54) **VERFAHREN UND VORRICHTUNG FÜR DIE -I(IN-VITRO)-KULTUR VON PFLANZEN-ZELLEN UND -ORGANEN SOWIE FÜR DIE MIKROPROPAGATION UND DIE REGENERATION VON GANZEN PFLANZEN.**

(30) Priorität: 15.02.85 CH 693/85

(43) Veröffentlichungstag der Anmeldung:
01.04.87 Patentblatt 87/14

(45) Bekanntmachung des Hinweises auf die Patenterteilung:
18.10.89 Patentblatt 89/42

(84) Benannte Vertragsstaaten:
AT BE CH DE FR GB IT LI LU NL SE

(56) Entgegenhaltungen:
EP-A- 0 017 650
EP-A- 0 052 001
DE-A- 2 037 013
FR-A- 2 522 014
GB-A- 2 123 706

(73) Patentinhaber: AGROGEN-STIFTUNG, Planche Supérieure 8, CH-1700 Fribourg (CH)

(72) Erfinder: KNOPF, Ulrich, C., Planche Supérieure 8, CH-1700 Fribourg (CH)

(74) Vertreter: Gassmann, Hans Ulrich, Dr., route J, Chaley 56, CH-1700 Fribourg (CH)

## Beschreibung

Methoden für die in-vitro-Kultur von Pflanzenzellen und Pflanzenorganen sowie für die Mikropropagation und Regeneration in vitro von ganzen Pflanzen werden heute in verschiedenen Laboratorien verwendet, um Zier- Nutz- und Medizinalpflanzen zu vermehren oder auf diesem Wege zu verbessern. In vitro kultivierte Zellen werden zudem für die Produktion von pflanzlichen Metaboliten (Sekundärstoffen), für Biotransformationen (Umwandlung von chemischen Stoffen unter Benützung lebender Zellen) und zum Testen verschiedener Agrochemikalien (Herbizide, Fungizide, Anti-Virus-Stoffe u.s.w.) verwendet.

In den meisten Laboratorien erfolgt die in-vitro-Kultur von Pflanzenzellen oder -organen auf mehr oder weniger komplexen Nährböden, die mit Agar oder Agarose zu einem Gel verfestigt sind, oder aber z.B. als Suspensionskulturen in flüssigen Nährlösungen (vergl. dazu H.E. Street, Plant Tissue and Cell Culture, 2nd edition, Blackwell Scientific Publications, Oxford 1977).

Es wurde nun gefunden, dass mittels einer neuen Methode auf die Verfestigung des Nährmediums mit Agar oder Agarose verzichtet werden kann, wobei aber die Zellen — im Gegensatz zur Suspensionskultur — trotzdem hauptsächlich in der gasförmigen Phase kultiviert werden. Das neue Verfahren, das Gegenstand der vorliegenden Erfindung bildet, besteht darin, dass man auf die Oberfläche des flüssigen Nährmediums frei bewegliche schwimmende Kugeln aufbringt, und die Pflanzenbestandteile an der Oberfläche dieser schwimmenden Kugeln kultiviert.

Gegenüber den bekannten Verfahren hat das Verfahren gemäss der vorliegenden Erfindung die folgenden Vorteile:

a) die Verdampfung (bzw. Austrocknung) des Nährmediums wird dank der die Oberfläche bedeckenden Schwimmkugeln auf ein Minimum reduziert. Damit können die Transplationsintervalle beträchtlich erhöht werden. In gewissen Fällen fällt dieser Aufwand gänzlich dahin, indem das flüssige Nährmedium in den Kulturgefässen bei Bedarf einfach ergänzt werden kann;

b) das erfindungsgemässe Verfahren erweist sich als besonders günstig zum Anlegen von neuen Starter-Kulturen aus Samen und andern Pflanzenteilen. Die Infektion durch Mikroorganismen kann dabei stark reduziert werden;

c) das neue Verfahren eignet sich insbesondere auch zum Testen von Agrochemikalien, wie z.B. Herbiziden, Pestiziden etc. sowie für die Induktion von Resistenzen in Pflanzenzellen, z.B. gegenüber Chemikalien oder Toxinen biologischen Ursprungs. Mit dem neuen Verfahren können Chemikalien schrittweise und in zunehmender Konzentration zu einer bereits angesetzten Kultur hinzugefügt werden; arbeitsaufwendige Transplantationen der Kulturen werden dabei unnötig. Zudem wird die gleichmässige Verteilung der Chemikalien oder Toxine sehr vereinfacht;

d) wegen der Möglichkeit, die sonst notwendigen Transplantationsschritte in verschiedene Nährmedien zu reduzieren oder gänzlich zu eliminieren, eignet sich das neue Verfahren vor allem auch für die Mikropropagation von ganzen Pflanzen, sowie die Regeneration von ganzen Pflanzen aus Zellen;

e) infolge des verminderten Arbeitsaufwandes und des Verzichts auf Agar und ähnliche Nährbodenverfestiger ist das Verfahren preisgünstig. Die schwimmenden Kugeln können, nach Reinigung und gegebenenfalls Sterilisation, mehrmals verwendet werden;

f) das erfindungsgemässe Verfahren eignet sich für die kontinuierliche Massenkultur von Pflanzenzellen. Insbesondere bietet es eine interessante Alternative in den Fällen wo die Suspensionskultur von Zellen nicht möglich oder wenig produktiv ist. Die Alternative ist auch von besonderem Interesse für die Produktion von Sekundärstoffen oder für Biotransformationen durch pflanzliche Zellen.

Die schwimmenden Kugeln gemäss der Erfindung müssen naturgemäss leichter sein als die Nährlösung, auf welcher sie schwimmen. Da sie bei einer mehrmaligen Verwendung gegebenenfalls sterilisiert werden müssen, verwendet man mit Vorteil Kugeln oder Hohlkugeln aus Kunststoffen. Zu ihrer Herstellung eignen sich insbesondere Polymere wie Polypropylen, Polyäthylen, Polyisobutylen oder Polystyrol.

Neben dem Verfahren umfasst die vorliegende Erfindung auch die zu dessen Ausübung geeigneten Vorrichtungen, d.h. die zur Aufnahme der Nährflüssigkeit und der Schwimmkugeln geeigneten Kulturgefässe, die mit zusätzlichen Einrichtungen für den Zu- und Wegtransport von Flüssigkeiten oder Gasen, für deren Zirkulation im Gefäss, für die Regelung physikalischer und chemischer Grössen, wie Licht, Temperatur, Druck, Flüssigkeitsniveau oder $p_H$ sowie gegebenenfalls mit einer räumlichen Abgrebzung für die schwimmenden Kugeln ausgerüstet sein können.

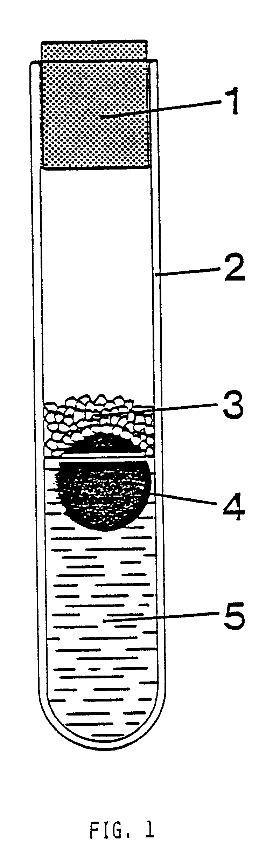

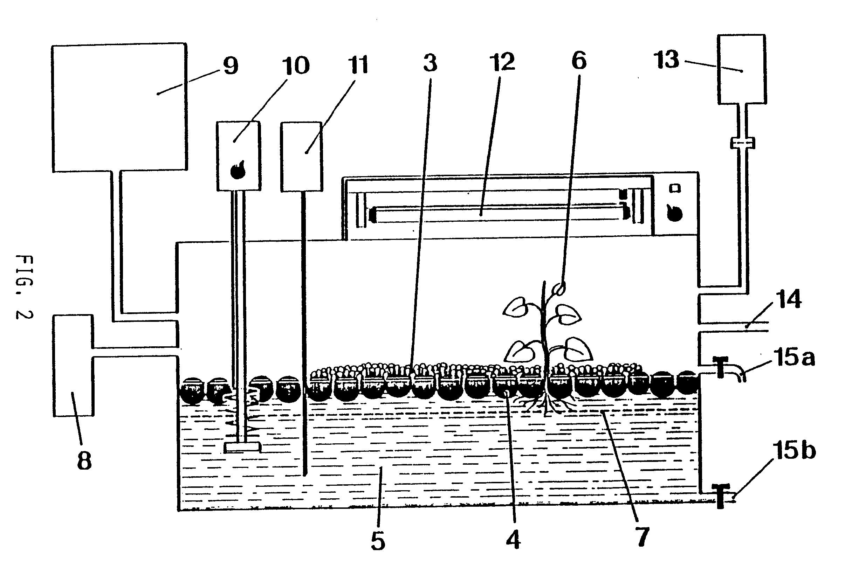

Die erfindungsgemässen Vorrichtungen werden durch die beiden Figuren 1 und 2 erläutert, ohne dass dadurch die möglichen Ausführungsformen in irgendwelcher Weise eingeschränkt werden sollen.

Figur 1 zeigt eine erfindungsgemässe Vorrichtung in ihrer einfachsten Ausführungsform: Das Kulturgefäss (2) besitzt die Form eines Reagensglases und ist mittels eines Stopfens (1) aus Kunststoff oder Baumwollwatte verschlossen. Im Gefäss befindet sich die Nährflüssigkeit (5) auf welcher eine Kugel aus Polypropylen (4) lose beweglich schwimmt. Auf der Oberfläche dieser Schwimmkugel entwickelt sich die Kultur (3) von Pflanzenzellen.

Figur 2 zeigt ein mit verschiedenen Zusatzvorrichtungen ausgerüstetes Kulturgefäss wie es sich für die Kultur in grösserem Massstab von Pflanzenzellen und -organen, sowie von ganzen Pflanzen eignet. In dem geschlossenen oder mit einem (in der Figur nicht gezeigten) keimdichten Deckel verschliessbaren Kulturgefäss befindet sich, dieses etwa zur Hälfte füllend, die Nährflüssigkeit (5), deren Oberfläche von einer sich zweidimensional erstreckenden Schicht schwimmender Kugeln (4) aus polypropylen praktisch vollständig bedeckt ist. Das Kulturgefäss ist mit einem Zulauf (9) und zwei verschiedenen Abläufen für die Nährflüssigkeit ausgerüstet, wovon der eine (15a) auf der Höhe der Flüssigkeitsoberfläche, der andere (15b) am Boden des Gefässes angebracht ist. Eine Rührvorrichtung (10) mit kombinierter Temperaturregelung und eine Vorrichtung zur Regelung des pH (11) sind in die Nährflüssigkeit eingetaucht. Aus dem Gefäss (13) kann ein Gas, z.B. sterile Luft in den freien Raum über der Nährflüssigkeit zugeführt werden. Der Stutzen (14) dient zur Ableitung des durch die Vorrichtung (13) eingeführten Gases, oder auch des durch die Vorrichtung (8) eingeführten Sterilisationsmittels (z.B. Dampf oder heisse Luft). Ein Gitter (7) dient dazu, die Schwimmkugeln auf der Flüssigkeitsoberfläche zu halten und, wenn nötig, zu bewegen. Mittels der Beleuchtungsvorrichtung (12) können die sich auf den Kugeln vermehrenden Zellkulturen (2) oder ganzen Pflanzen (6) nach Bedarf bestrahlt werden.

Für die in den nachfolgenden Beispielen beschriebenen Versuche wurden Kulturgefässe aus Glas, verschlossen mit Baumwolle- oder Kunststoffstopfen, bzw. bei grösseren Gefässen mit einem Deckel aus Kunststoff oder Aluminium, verwendet. Die Gefässe wurden vor Beginn der Versuche mit heissem Dampf (110°C, 1 atü) sterilisiert. Zur Sterilisation können gegebenenfalls auch radioaktive Strahlen, z.B. -Strahlen verwendet werden. Den sterilisierten Gefässen wurde anschliessend flüssige Nährlösung zugegeben (für die Zusammensetzung geeigneter Nährlösungen siehe z.B. O.L. Gamborg, T.A. Murashige, A. Thorpe und K. Vasil, Plant Tissue Culture Media, In Vitro 12, 473-478, 1976). Die zu kultivierenden, gegebenenfalls zuvor sterilisierten Pflanzenzellen oder -organe (inkl. Samen) wurden anschliessend auf die schwimmenden Kugeln aufgebracht.

Als biologische Materialien für die nachfolgenden Beispiele verwendeten wir in vitro kultivierte Zellen (Kalli) und Organe der Luzerne (Medicago sativa), des Tabaks (Nicotiana tabacum), der kartoffel (Solanum tuberosum), des Blumenkohls (Brassica oleracea) und des Weizens (Triticum aestivum). Das erfindungsgemässe System eignet sich jedoch grundsätzlich für Zellkulturen sämtlicher Pflanzenarten. Die Kulturen wurden Während mehrerer Wochen bzw. Monaten bei 28°C unter Kunstlicht mit verschiedenen Photoperioden oder auch in der Dunkelheit kultiviert.

*Beispiel 1*

Anlage von neuen Zellkulturen.

Verschiedene Gewebe und Organe inkl. Samen der oben genannten Pflanzen wurden wie folgt sterilisiert:

1-3 min in 70%igem Äthanol
1 min in destill. Wasser
10-20 min in Na-Hypochlorit, 5%ige wäss. Lösung, gefolgt von mehrmaligem Waschen mit sterilem dest. Wasser.

In Reagensläsern von 10 cm Länge und 11 mm Durchmesser wurden die Gewebe auf einer Polypropylenkugel von 10 mm Durchmesser kultiviert, die auf 3 ml der folgenden Nährlösung schwamm:

Nährlösung B5, (vergl. Gamborg et al. In Vitro 12, 473-478, 1976), enthaltend pro Liter

| 2500 | mg | $KNO_3$ |
|---|---|---|
| 150 | mg | $CaCl_2 \cdot 2H_2O$ |
| 250 | mg | $MgSO_4 \cdot 7H_2O$ |
| 134 | mg | $(NH4)_2SO_4$ |
| 150 | mg | $NaH_2PO_4 \cdot H_2O$ |
| 0.75 | mg | KI |
| 3.0 | mg | $H_3BO_3$ |
| 10 | mg | $MnSO_4 \cdot H_2O$ |
| 2 | mg | $ZnSO_4 \cdot 7H_2O$ |
| 0.25 | mg | $Na_2MoO_4 \cdot 2H_2O$ |
| 0.025 | mg | $CuSO_4 \cdot 5H_2O$ |
| 0.025 | mg | $CoCl_2 \cdot 6H_2O$ |
| 37.3 | mg | $Na_2EDTA$ *) |
| 27.8 | mg | $FeSO_4 \cdot 7H_2O$ |
| 20 | g | Saccharose |
| 100 | mg | Inosit |
| 1 | mg | Nicotinsäure |
| 1 | mg | Pyridoxin-HCl |
| 10 | mg | Thiamin-HCl |
| 1 | mg | 2,4-D **) |

pH der Lösung eingestellt auf 5.5

*) Dinatriumsalz der Äthylendiamin-Tetraessigsäure
**) 2,4-Dichlor-phenoxyessigsäure

Die Kultur wurde so lange fortgesetzt bis eine deutliche Kallusbildung oder eine Keimung der Samen erfolgte.

*Beispiel 2*

Erhaltung von Zellkulturen und Mikropropagation von Pflanzen.

Versuche mit dem gleichen Pflanzenmaterial und der gleichen Nährlösung wurden in Reagensgläsern von 16 cm Länge und 25 mm Weite durchgeführt, wobei eine Polypropylenkugel von 25 mm Durchmesser verwendet wurde. Den Kulturen wurde, wo nötig, von Zeit zu Zeit frische Nährlösung zugefügt.

*Beispiel 3*

Testen von Chemikalien (Herbizide, Pestizide etc.) Induktion von Resistenzen gegen Chemikalien und Toxine.

Zellkulturen wurden angelegt wie in den Beispielen 1 und 2 beschrieben und für einige Zeit erhalten. Nachdem die Kalli eine gewisse Grösse erreicht hatten, wurden die Chemikalien, z.B. Herbizide, in regelmässigen Zeitabständen und zunehmender Konzentration zugegeben. Die überlebenden Kalli wurden selektioniert. Wo nötig wurde den Kulturen von Zeit zu Zeit frische Nährlösung zugefügt, ohne dass die Kalli dabei transplantiert werden mussten.

*Beispiel 4*

Regeneration von Pflanzen aus Zellen und Organen.

Kalli wurden wie im Beispiel 2 beschrieben, oder aber in Glasgefässen von 5 cm Durchmesser und variabler Höhe, welche eine grössere Anzahl auf der Nährlösung schwimmender Polypropylenkugeln enthielten, kultiviert. Um eine Regeneration von ganzen Pflanzen zu bewirken, wurden entweder die dazu notwendigen Chemikalien direkt ins Kulturgefäss zugegeben, oder die dazu nötige neue Nährlösung wurde mit oder ohne Absaugen der alten Lösung in die Kulturgefässe gefüllt.

*Beispiel 5*

Kontinuierliche Kultur von Pflanzenzellen.

Für die kontinuierliche Kultur von Pflanzenzellen wurde eine Vorrichtung entsprechend der Figur 2 verwendet. Die Nährlösung wurde kontinuierlich oder periodisch abgesaugt und durch frische Lösung ersetzt, ohne dabei das Niveau des Nährmediums und der darauf schwimmenden Kulturen zu verändern.

## Patentansprüche

1. Verfahren für die in-vitro-Kultur von Pflanzenzellen und -organen sowie für die Mikropropagation und Regeneration in vitro von ganzen Pflanzen, dadurch gekennzeichnet, dass die Kultur auf an der Oberfläche eines flüssigen Nährmediums schwimmenden Kugeln aus einem polymeren Kunststoff erfolgt.

2. Vorrichtung zur Durchführung des verfahrens gemäss Anspruch 1, dadurch gekennzeichnet, dass sie aus einem vorzugsweise sterilisierbaren Gefäss zur Aufnahme der Nährlösung und der darauf schwimmenden Kugeln besteht.

3. Vorrichtung gemäss Anspruch 2, dadurch gekennzeichnet, dass das Gefäss mit mindestens einem Zu- (9) und Ablauf (15a, 15b) für Flüssigkeit ausgerüstet ist, die ober- oder unterhalb oder auf gleicher Höhe wie das Flüssigkeitsniveau am Gefäss angebracht sind.

4. Vorrichtung gemäss einem der Ansprüche 2 oder 3, dadurch gekennzeichnet, dass das Gefäss zusätzlich mit Vorrichtungen für die Zufuhr (13) oder Abfuhr (14) eines Gases ausgerüstet ist.

5. Vorrichtung gemäss einem der Ansprüche 2, 3 oder 4, dadurch gekennzeichnet, dass das Gefäss zusätzlich eine Rührvorrichtung enthält.

6. Vorrichtung gemäss einem der Ansprüche 2 bis 5, dadurch gekennzeichnet, dass das Gefäss zusätzlich mit Vorrichtungen zur Regulierung der Temperatur (10) und/oder des $p_H$ (11) ausgerüstet ist.

7. Vorrichtung gemäss einem der Ansprüche 2 bis 6, dadurch gekennzeichnet, dass das Gefäss unterhalb des von den schwimmenden Kugeln beanspruchten Niveaus mit einem für die letzteren nicht durchlässigen Gitter (7) ausgerüstet ist.

8. Vorrichtung gemäss einem der Ansprüche 2 bis 7, dadurch gekennzeichnet, dass das Gefäss zusätzlich noch mit einer auf die Kultur wirkenden Beleuchtungsvorrichtung (12) ausgerüstet ist.

9. Vorrichtung gemäss einem der Ansprüche 2 bis 8, dadurch gekennzeichnet, dass das Gefäss zusätzlich mit einer Vorrichtung (8) für die Sterilisation ausgerüstet ist.

## Claims

1. Process for the in vitro culture of plant cells and organs as well as for the micropropagation and regeneration in vitro of whole plants characterized in that the culture is established on spheres of a polymer plastic material which are floating on the surface of a liquid nutrient medium.

2. Apparatus for the realization of the process according to claim 1, characterized in that it consists of a vessel in which to receive the nutrient liquid and the spheres, said vessel preferably being sterilizable.

3. Apparatus according to claim 2, characterized in that the vessel is at least provided with an inlet (9) and an outlet (15a, 15b) for liquid, which ate positioned either above or below, or then at the same height as the level of the liquid surface.

4. Apparatus according to one of the claims 2 or 3, characterized in that the vessel is additionally provided with devices for admitting (13) and carrying off (14) a gas.

5. Apparatus according to one of the claims 2, 3 or 4, characterized in that the vessel is additionally provided with a stirring device.

6. Apparatus according to one of the claims 2 to 5, characterized in that the vessel is additionally provided with devices for regulating the temperature and/or the $p_H$.

7. Apparatus according to one of the claims 2 to 6, characterized in that the vessel, below the level of the floating spheres, is provided with a grid, which is impermeable to the latter.

8. Apparatus according to one of the claims 2 to 7, characterized in that the vessel is provided with an additional device for illumination.

9. Apparatus according to one of the claims 2 to 8, characterized in that the vessel is additionally provided with a device for sterilization.

## Revendications

1. Procédé pour la culture in vitro de cellules et organes de plantes et pour la micropropagation et régénération in vitro de plantes entières, caractérisé en ce que ladite culture est faite sur des sphères en matière plastique polymère qui flottent sur la surface d'un bouillon de culture.

2. Appareil pour la mise en œuvre du procédé selon la revendication 1, caractérisé en ce qu'il consiste en un récipient pour l'accueil du bouillon et des sphères flottant sur ce dernier, ledit récipient étant, de préférence, stérilisable.

3. Appareil selon la revendication 2, caractérisé en ce que le récipient est muni au moins de dispositifs pour l'alimentation (9) et l'écoulement (15a, 15b) d'un liquide, lesdits dispositifs étant placés au-dessus, au-dessous ou à la même hauteur que le niveau du liquide dans le récipient.

4. Appareil selon l'une des revendications 2 ou 3, caractérisé en ce que le récipient est muni de conduites supplémentaires pour l'amenée (13) et l'enlèvement (14) d'un gaz.

5. Appareil selon une des revendications 2, 3 ou 4, caractérisé en ce que le récipient est muni d'un agitateur supplémentaire.

6. Appareil selon une des revendications 2 à 5, caractérisé en ce que le récipient est muni de dispositifs supplémentaires pour le réglage de la température (10) et/ou du pH (11).

7. Appareil selon une des revendications 2 à 6, caractérisé en ce que le récipient, au-dessous du niveau occupé par les sphères, est muni d'une grille (7) non perméable aux sphères.

8. Appareil selon une des revendications 2 à 6, caractérisé en ce que le récipient est muni d'un dispositif supplémentaire d'illumination (12).

9. Appareil selon une des revendications 2 à 8, caractérisé en ce que le récipient est muni d'un dispositif supplémentaire pour la stérilisation (8).

FIG. 1

FIG. 2